# EUROPÄISCHE PATENTSCHRIFT

(11) **EP 2 249 692 B1**
(45) Veröffentlichungstag und Bekanntmachung des Hinweises auf die Patenterteilung: **17.12.2014**
(21) Anmeldenummer: 09703661.0
(22) Anmeldetag: 23.01.2009
(51) Int. Cl.: A61B 3/12

(54) **FUNDUSKAMERAOBJEKTIV UND KAMERA MIT FUNDUSKAMERAOBJEKTIV**
FUNDUS CAMERA OBJECTIVE AND CAMERA HAVING FUNDUS CAMERA OBJECTIVE
OBJECTIF DE CAMÉRA DE FOND D'OEIL ET CAMÉRA POURVUE DE CET OBJECTIF

(30) Priorität: 24.01.2008 AT 992008
(43) Veröffentlichungstag der Anmeldung: 17.11.2010
(73) Patentinhaber: S & V Technologies GmbH, 16761 Hennigsdorf (DE)
(72) Erfinder: BÄR, Leopold, A-2392 Sulz im Wienerwald (AT); FROMBERG, Ingeborg, 16761 Hennigsdorf (DE)
(74) Vertreter: Ter Meer Steinmeister & Partner
(86) Internationale Anmeldenummer: PCT/EP2009/000429
(87) Internationale Veröffentlichungsnummer: WO 2009/092598

(56) Entgegenhaltungen:
- WO-A-2004/017825
- WO-A-2005/122874
- DE-U1-202006 000 562
- US-A1- 2005 041 207
- US-A1- 2005 200 707

## Beschreibung

Die Erfindung betrifft ein Funduskameraobjektiv zur Aufnahme des Hintergrundes eines Auges. Weiter betrifft die Erfindung eine Kamera mit einem Funduskameraobjektiv. Darüber hinaus betrifft die Erfindung ein Ringlicht, das mit dem Funduskameraobjektiv oder einer Kamera verbunden wird und der Beleuchtung der Umgebung des Auges dient.

Mit einer Funduskamera kann eine präzise Aufnahme des Augenhintergrundes aufgenommen werden, bei der insbesondere die Netzhaut, Blutgefäße, der Sehnerv und die Aderhaut darstellbar ist. Die Funduskamera wird zur der Diagnostik von Veränderungen des Auges und zur Verfolgung von Krankheitsverläufen eingesetzt. Herkömmliche Funduskameras werden von verschiedenen Herstellern angeboten und sind als Spezialkameras mit einem Spezialobjektiv ausgebildet. Kamera und Objektiv sind dabei in einem gemeinsamen Gehäuse angeordnet und über Verbindungskabel mit einer Bildverarbeitungseinheit gekoppelt.

Ein erster Nachteil der herkömmlichen Funduskameras besteht darin, dass diese über Kabel und Leitungen mit einer bildverarbeitenden Basisstation verbunden sind. Darüber hinaus haben herkömmliche Funduskameras ein hohes Eigengewicht. Ein weiterer Nachteil besteht darin, dass die Bestandteile der Kamera nicht durch den Endanwender austauschbar sind und somit nicht an Weiterentwicklungen oder Neuerungen bzw. unterschiedliche Anwendungsbereiche angepasst werden können. Bei herkömmlichen Funduskameras können weder das Objektiv noch Bestandteile des Objektivs ausgetauscht werden, noch kann die aufnehmende Kamera in ihrer Bildauflösung. Speicherkapazität oder Bildverarbeitungs-funktionalität verändert werden. Die herkömmlichen Geräte sind meist sehr teuer, da die Stückzahlen gering sind.

Herkömmliche Digitalkameras haben in den letzten Jahren eine rasante Entwicklung erfahren, insbesondere im Auflösungsbereich und in der Bildverarbeitungsfunktionalltät.

Bisher sind keine Funduskameraobjektive bekannt, die separat erhältlich sind und an verschiedene Analog - bzw. Digitalkameras anschließbar sind.

US 2005/0041207 beschreibt ein Beleuchtungssystem zur Untersuchungen eines Auges. Ein optisches System mit einer Koppellinse und einem Objektiv ist an eine Bildaufnahmeeinheit angeschlossen. Das optische System enthält zwischen Koppellinse und Objektiv einen Lichtemitter.

WO 2005/122874 beschreibt eine ophthalmische Kamera und einen Kameraadapter, der an eine Kamera angeschlossen ist. Der Kameraadapter weist ein starres Gehäuse auf, welches einen Schnittstellenkontakt aufweist.

WO 2004/017825 A1 beschreibt eine portable ophthalmische Vorrichtung, die eine Abstützeinheit aufweist, in die ein Mobilfunktelefon mit einer Kamera lösbar eingesetzt ist. Die Abstützeinheit mit dem Mobiltelefon und der Kamera ist mit einer ophthalmischen Vorrichtung verbunden ist, die eine entsprechende Optik und ein Beleuchtungssystem aufweist.

Daher ergibt sich die Aufgabe, ein Funduskameraobjektiv und eine Kamera anzugeben, wobei das Funduskameraobjektiv an eine allgemeine Analog- oder Digitalkamera anschließbar ist, wobei einzelne Komponenten austauschbar sind, und somit eine einfache Anpassung an modernere Kameras oder andere Einsatzzwecke möglich ist.

Die Aufgabe wird durch ein Funduskameraobjektiv zur Aufnahme des Hintergrundes eines Auges gemäß Anspruch 1 gelöst.

Kern der Erfindung ist ein Funduskameraobjektiv mit einer Funduskameraobjektivanschlusseinheit, die eine Verbindung mit einer Analog- bzw. Digitalkamera ermöglicht. Das Funduskameraobjektiv weist die üblichen optischen Komponenten wie Objektiv, Tubus und Okular auf. Die Funduskameraobjektivanschlusseinheit ist so ausgebildet, dass sie an herkömmliche Analog- bzw. Digitalkameras anschließbar ist. Dadurch wird es dem Benutzer, insbesondere dem Ophthalmologen ermöglicht, ein Funduskameraobjektiv zu erwerben und dieses über einen längeren Zeitraum zu nutzen, wobei die als Funduskamera verwendete Kamera häufiger ersetzt werden kann, wodurch eine Anpassung an den jeweiligen Entwicklungsstand derartiger Kameras möglich wird.

Der Tubus des Funduskameraobjektivs verstellbar, wodurch sich der Abstand zwischen Objektiv und Okular einstellen lässt, um somit eine Anpassung an unterschiedliche Anwendungen zu ermöglichen.

Vorzugsweise ist zur Beleuchtung des aufzunehmenden Hintergrundes eines Auges und/oder der Umgebung ein Ringlicht vorgesehen, welches im vorderen Bereich des Funduskameraobjektives angeordnet ist und mit einer externen Lichtquelle gekoppelt ist, die dem Ringlicht Licht zuführt. Das Ringlicht kann mit einem Strahlenteiler verbunden werden, in den Licht einer externen Lichtquelle und/oder eines Blitzlichtes eingekoppelt wird. Alternativ ist es möglich, dass das Ringlicht an eine Beleuchtungseinheit angeschlossen wird, die sowohl ein Licht einer vorbestimmten Wellenlänge, als auch ein Blitzlicht abgeben kann. Bei Einsatz einer derartigen Beleuchtungseinheit ist ein Strahlenteiler nicht erforderlich. Der Strahlenteiler weist vorzugsweise zwei Eingänge und wenigstens einen Ausgang aus, wobei an dem einen Eingang eine externe Lichtquelle angeschlossen wird und an dem anderen Eingang ein Blitzlicht angeschlossen wird. Als Blitzlicht kann vorzugsweise das Blitzlicht der Kamera verwendet werden. Der wenigstens eine Ausgang des Strahlenteilers ist mit dem Ringlicht koppelbar. Am Eingang des Strahlenteilers, der mit dem Blitzlicht verbunden ist, ist ein Strahlenumformer vorhanden, der den Querschnitt des Blitzlichtes der Kamera bzw. einer separaten Blitzröhre an den Querschnitt des Strahlenteilers anpasst.

Weiter ist vorgesehen, dass Funduskameraobjektiv mit einem Handgriff zu verbinden, der es ermöglicht, das Funduskameraobjektiv einhändig zu bedienen. In diesem Handgriff können vorzugsweise Batterien oder Akkumulatoren vorhanden sein, die eine Energieversorgung bereitstellen, beispielsweise für die Kamera, für ein externes Blitzlicht und/oder für die externe Lichtquelle für das Ringlicht. Der Handgriff weist vorzugsweise einen Auslöser auf, der ein Auslösesignal über einen mechanischen Drahtauslöser, eine elektrische Leitung oder über Funk zur Kamera überträgt und dort eine Aufnahme auslöst.

Vorzugsweise ist das Funduskameraobjektiv mit einer Umkehroptik ausgestattet, die ein aufgenommenes Bild umdreht, um somit ein aufrechtes Bild zu erhalten. Die Umkehroptik ist bei Anschluss einer Digitalkamera nicht zwingend erforderlich, da eine spätere Bildverarbeitung eine automatische Bildumkehr zur Darstellung eines aufrechten Bildes bereitstellen kann.

Vorzugsweise hat das Funduskameraobjektiv eine Einstellvorrichtung zum Dioptrienausgleich, der durch Verschieben von Objektiv und Okular zueinander erreicht wird. Die ausgeglichenen Dioptrien sind auf einer Skala an der Außenseite des Tubus ablesbar.

Weiter kann das Funduskameraobjektiv eine Feldblende aufweisen, die in den Strahlengang des Funduskameraobjektivs eingesetzt ist und beispielsweise in Form einer Irisblende ausgestaltet ist. Mit Hilfe diese Feldblende kann der Durchmesser des Strahlengangs verändert werden. Der Durchmesser der Feldblende ist über einen am Außenumfang des Funduskameraobjektives angeordneten Blendeneinstellring verstellbar.

Alternativ zu einer verstellbaren Feldblende ist es jedoch auch möglich, eine Feldblende mit einer fixen Blendenweite einzusetzen. Der Tubus des Funduskameraobjektivs kann weiter Filter aufweisen, die beispielsweise eine Farbfilterung vornehmen, um das Funduskameraobjektiv für verschiedene Anwendungen bei der Untersuchung des Auges anzupassen.

Bei einer vorteilhaften Ausgestaltung kann das Funduskameraobjektiv zur Aufnahme so ausgestaltet werden, dass eine Anglographieaufnahme des Augenhintergrundes möglich wird. Zur fotographischen Darstellung der Blutgefäße der Netzhaut wird ein Kontrastmittel zum Beispiel Floureszein bspw. in die Arm oder Vorderbeinvene gespritzt. Das Funduskameraobjektiv erfordert für eine Anglographieaufnahme einen Erregerfilter, der zugeführtes weißes Licht filtert, so dass nur blaues Licht mit einer Wellenlänge von ca. 480 nm auf die Netzhaut trifft. Die Floureszein Moleküle werden dabei angeregt und geben grünes Licht ab. Es wird aber auch blaues Licht reflektiert und zum Strahlengang des Funduskameraobjektivs geleitet. Dort ist ein Sperrfilter angeordnet, der den blauen Anteil des Lichts herausfiltert, so dass nur ein grüner Lichtanteil mit einer Wellenlänge von 510 bis 530 nm zur Funduskamera hindurch dringen kann, wodurch eine Angiographieaufnahme der Netzhaut ermöglicht wird.

Das Ringlicht ist vorzugsweise über einen Lichtleiter mit dem Strahlenteiler oder mit einer externen Lichtquelle oder mit einer Beleuchtungseinheit verbunden, wobei die dem Ringlicht zugeführte Lichtmenge einstellbar ist. Die Kameraanschlusseinheit ist vorzugsweise als Gewinde ausgestaltet oder weist einen Bajonettverschluss auf, so dass das Funduskameraobjektiv mit der Funduskameraobjektivanschlusseinheit an eine herkömmliche Analog- bzw. Digitalkamera anschließbar ist.

Darüber hinaus wird die Aufgabe auch durch eine Kamera mit einem oben beschriebenen Funduskameraobjektiv gelöst, wobei die Kamera eine Kameraanschlusseinheit aufweist, die einen Anschluss des Funduskameraobjektivs und insbesondere der Funduskameraobjektivanschlusseinheit ermöglicht. Die Kameraanschlusseinheit weist somit vorzugsweise ebenso ein korrespondierendes Gewinde auf oder ist so ausgebildet, dass ein Funduskameraobjektiv mit einem Bajonettverschluss an die Kamera anschließbar ist.

Die als Funduskamera eingesetzte Kamera kann eine herkömmliche Analog- bzw. Digitalkamera sein und weist somit ein Display, einen Speicher beispielsweise in Form einer Speicherkarte und einen Bildprozessor auf, der eine Bildaufbereitung ermöglicht.

Der oben beschriebene Handgriff kann alternativ zur Befestigung am Funduskameraobjektiv auch direkt an der Funduskamera befestigt werden, wobei auch in diesem Fall sowohl die Kamera als auch das Ringlicht bzw. die externe Lichtquelle und/oder das Blitzlicht über Akkus bzw. Batterien im Handgriff mit Energie versorgt werden können.

Vorzugsweise kann der Handgriff unabhängig vom Ort seiner Befestigung eine Lichtquelle bzw. Beleuchtungseinheit enthalten, deren Licht entweder in den Strahlenteiler einkoppelbar ist, bzw. direkt dem Ringlicht zuführbar ist. Somit ist es möglich, in den Handgriff eine Lichtquelle zu Integrieren, die unmittelbar über die Batterien oder Akkumulatoren im Handgriff mit Energie versorgt wird, deren Lichtstärke unmittelbar am Handgriff einstellbar ist, wobei somit nur noch eine Zuleitung des Lichts beispielsweise über einen Lichtleiter zum Ringlicht erforderlich ist.

Ein weiterer Aspekt der Erfindung betrifft das Ringlicht, das eine bessere Ausleuchtung von Makroaufnahmen ermöglicht, wobei das Ringlicht sein Licht in Richtung Auge abstrahlt, und somit das Auge ausleuchtet. Das Ringlicht kann vorzugsweise über einen Lichtleiter mit einer externen Lichtquelle gekoppelt sein, oder auch an ein Blitzlicht angeschlossen sein. Alternativ ist es möglich, das Ringlicht mit dem zuvor beschriebenen Strahlenteiler zu koppeln, an den eine externe Lichtquelle und ein Blitzlicht anschließbar sind. Das Ringlicht kann auch ohne Funduskameraobjektiv verwendet werden und an eine normale Analog- bzw. Digitalkamera angeschlossen werden, um eine bessere Ausleuchtung bei Makroaufnahmen zu ermöglichen.

Im Folgenden wird die Erfindung anhand der Figuren näher erläutert.
Fig. 1 zeigt einen Aufbau eines erfindungsgemäßen Funduskameraobjektivs, das an eine Funduskamera angeschlossen ist:
Fig. 2 zeigt eine alternative Ausgestaltung eines erfindungsgemäßen Funduskameraobjektivs. das an eine Funduskamera angeschlossen ist;
Fig. 3 zeigt eine Kamera, die mit einem erfindungsgemäßen Ringlicht gekoppelt ist und von einem erfindungsgemäßen Handgriff abgestützt wird.

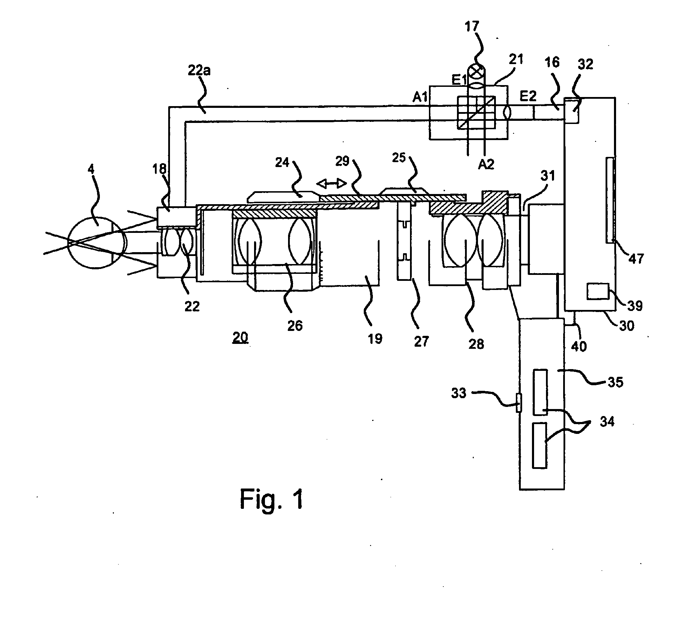

Gemäß Fig. 1 ist das erfindungsgemäße Funduskameraobjektiv 20 an eine digitale oder analoge Kamera 30 angeschlossen. Dazu weist das Funduskameraobjektiv 20 eine Funduskameraobjektivanschlusseinheit 31 auf, die mit einer Kameraanschlusseinheit 36 koppelbar ist. Die Kameraanschlusseinheit 36 ist beispielsweise als Innengewinde ausgestaltet, wobei die Funduskameraobjektivanschluss 31 ein Außengewinde aufweist. Alternativ ist ein Bajonettverschluss zwischen Funduskameraobjektivanschluss 31 und Kameraanschlusseinheit 36 möglich.

Die Netzhaut des Auges 4 wird mit Licht bestrahlt, das von dem Ringlicht 18 abgestrahlt wird. Die von der Netzhaut des Auges 4 kommenden Lichtstrahlen verlassen das Auge und werden dem Funduskameraobjektiv zugeführt und treffen dort parallel auf ein Objektiv 22. Der Strahlengang verläuft innerhalb des Tubus 19 des Funduskameraobjektivs 20 und verlässt das Funduskameraobjektiv über das kameraseitig angeordnete Okular 28, um auf einen Film in einer Analogkamera oder einen lichtempfindlichen Sensor, bspw. einen CCD-Sensor, in einer Digitalkamera aufzutreffen. Die Kamera 20 ist dabei in ihrer Brennweite vorzugsweise auf "unendlich" eingestellt. Das Bild wird auf dem Bildaufnahmemedium gespeichert und für eine weitere Auswertung der Aufnahme bereitgehalten. Bei einer Digitalkamera ist dazu ein Signalprozessor vorgesehen, der eine Bildverarbeitung vornehmen kann und das auf dem CCD-Sensor gespeicherte Bild zu einem nichtflüchtigen Speicher, bspw. in Form einer Speicherkarte, überträgt. Bei einer Analogkamera muss der Film entwickelt werden.

Das Funduskameraobjektiv 20 enthält vorzugsweise eine Umkehroptik 26, um ein aufrechtes Bild des Auges entstehen zu lassen, wobei die Umkehroptik 26 bei einer Digitalkamera jedoch auch entfallen kann, da das Bild hier elektronisch gedreht werden kann. Zum Ausgleich einer eventuellen Fehlsichtigkeit des Auges ist ein Dioptrieausgleich vorgesehen, der sich durch einen Einstellring 24 realisieren lässt. Dazu ist eine Skala 29 auf der äußeren Seite des Tubus 19 angeordnet, die ein Ablesen der ausgeglichenen Dioptrien ermöglicht. Der Dioptrieausgleich ist erforderlich, um die Strahlen, die das Funduskameraobjektiv verlassen, parallel auszurichten. Durch Drehen des Einstellringes 24 werden die Linsen des Objektivs 22 und des Okulars 28 innerhalb des Funduskameraobjektives 20 zu einander verschoben.

Das Funduskameraobjektiv 20 weist weiter eine Feldblende 27 auf, die als Irisblende ausgestaltet ist, wobei durch Drehen eines Einstellringes 25 der Durchmesser der Blende verstellbar ist. Alternativ kann die Blende 27 auch als fixe Blende ausgestaltet sein. Der Tubus 19 kann an verschiedenen Stellen innerhalb des Strahlengangs Filter aufweisen. Ebenso ist es möglich, vor das Objektiv 22 des Funduskameraobjektivs einen austauschbaren Aufsetzfilter anzuordnen.

Zur Beleuchtung des aufzunehmenden Auges ist ein faseroptisches Ringlicht 18 vorgesehen, das im vorderen Bereich des Funduskameraobjektivs 20 montiert ist und Licht in Richtung Auge abstrahlt. Das Ringlicht 18 kann dabei verschiedene Durchmesser, beispielsweise 2,5 cm, aufweisen und kann somit an verschiedene Anwendungen und an verschiedene Patienten angepasst werden (Kinder, Erwachsene). Das Ringlicht 18 ist über ein Lichtleiterkabel 22a mit einem Strahlenteiler 21 verbunden. An dem Strahlenteiler 21 ist vorzugsweise eine externe Lichtquelle 17 angeschlossen, die dimmbar ausgestaltet ist. Weiter ist an den Strahlenteilern über einen Eingang E2 ein Blitzlicht 32 angeschlossen. Dabei kann das Blitzlicht 32 der Kamera 30 verwendet werden. Es kann jedoch auch ein externes Blitzlicht verwendet werden. Das Blitzlicht wird vorzugsweise über einen Strahlenumformer 16 in den Strahlenteiler 21 eingekoppelt.

Der Strahlenteiler 21 dient zum Vereinen des Lichtes der externen Lichtquelle 17 und des Blitzlichts 32. Lichtstrahlen, die an den beiden Eingängen E1 und E2 eintreten, werden im Strahlenteiler 21 vereint und treten am wenigstens einen Ausgang A1 wieder aus. Der zweite Ausgang A2 ist in Fig. 1 offen und kann zum Anschluss einer zweiten Beleuchtungsmöglichkeit, beispielsweise zur Umgebungsbeleuchtung benutzt werden. An den Ausgang 1 und ggf. auch an den Ausgang A2 wird jeweils ein Lichtkabel 22a angeschlossen, mit dem das vom Strahlenteiler 21 ausgegebene Licht zum Ringlicht 18 bzw. zur weiteren Beleuchtungsmöglichkeit geführt wird. Am Eingang E1 ist eine externe Lichtquelle 17 montiert. Ggf. kann eine Optik zur Fokussierung vorhanden sein. Ebenso ist es möglicht, das Licht bereits bei der Einkopplung vor dem Strahlenteiler 21 durch einen einsetzbaren Filter zu filtern.

Um die Funduskamera bzw. das Funduskameraobjektiv 20 auch non-mydriatisch, d. h. ohne die Pupille künstlich zu weiten, anwenden zu können, kann auch eine IR-Lichtquelle 17 angeschlossen werden. Damit die gesamte Lichtmenge der externen Lichtquelle 17 bzw. des Blitzlichts 32 in das Lichtleitkabel 22a eingekoppelt wird, bündelt eine jeweils am Eingang E1 bzw. E2 angeordnete optische Linse das Licht, so dass der gesamte Querschnitt des Lichtleiterkabels 22a beleuchtet ist. Der Strahlenumformer 16 am Eingang E2 besteht aus Lichtleitfasern, die so geformt sind, dass der Querschnitt auf der Seite zum Strahlenteiler 21 hin rund ist. Das andere Ende der Lichtleitfasern ist so ausgebildet, dass es dem Querschnitt des Kamerablitzes 32 entspricht und meist quadratisch ausgebildet ist. Der Blitz 32 kann in die Kamera 30 eingebaut sein. Es kann jedoch auch ein externes Blitzgerät verwendet werden. Der Strahlenumformer 16 ist dabei so vor dem jeweiligen Blitzlicht 32 montiert, dass das austretende Blitzlicht durch den Strahlenumformer 16 und den Strahlenteiler 21 weiter über das Lichtleitkabel 22a zum Ringlicht 18 geführt wird und somit eine Beleuchtung des Hintergrundes des Auges 4 ermöglicht. Im Strahlenteiler 21 werden der Beleuchtungsstrahl der externen Lichtquelle 17 und der Blitzlichtstrahl des Blitzlichts 32 in einen gemeinsamen Strahlengang eingekoppelt, wobei das Blitzlicht über diesen Weg zum Augenhintergrund gelangt, um diesen für den Zeitpunkt der Aufnahme ausreichend auszuleuchten. Um dabei möglichst viel Licht zum Auge zu leiten, ist es möglich, das Lichtleitkabel 22a an beide Ausgänge A1 und A2 anzuschließen und somit das Licht dem Ringlicht 18 zuzuführen. Alternativ ist es möglich, an den Ausgang A2 eine weitere Beleuchtungsquelle anzuschließen, um eine weitere Beleuchtung des aufzunehmenden Objekts zu realisieren. Wenn beide Ausgänge A1 und A2 an das Lichtleitkabel 22a angeschlossen werden, ist die Ausbeute des Lichts nahezu 100%, da an jedem Ausgang A1 und A2 des Strahlenteilers 21 nur 50% des einfallenden Lichts zur Verfügung stehen.

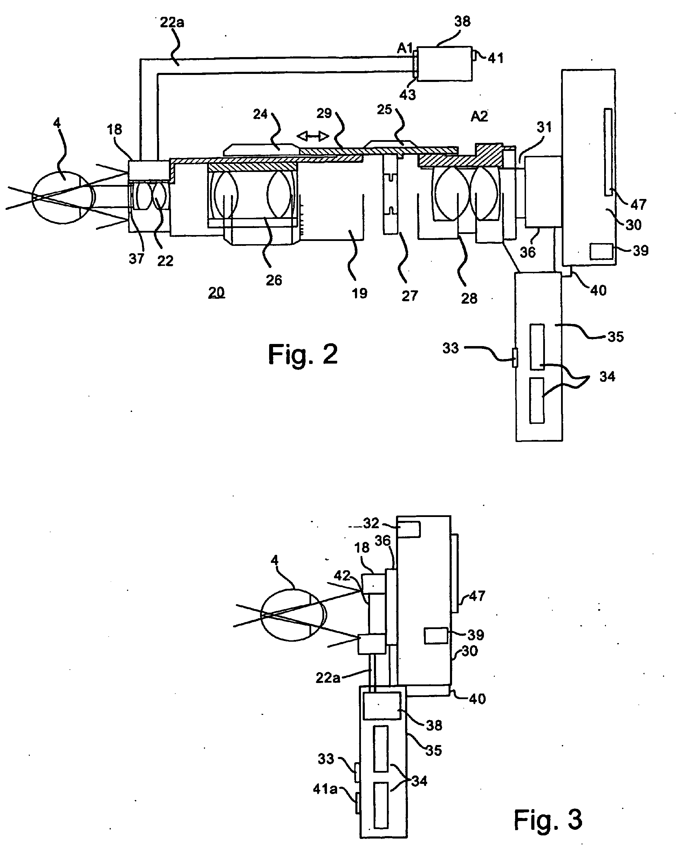

Über das Ringlicht 18 wird der Augenhintergrund des Auges 4 beleuchtet. Damit es nicht zu störenden Reflexen auf der Hornhaut kommt, sind der Beleuchtungs- und der Beobachtungsstrahlengang voneinander getrennt. Die Beleuchtung erfolgt seitlich am Beobachtungsstrahlengang vorbei. Die Lichtmenge, die von der externen Lichtquelle 17 bzw. von der Beleuchtungseinheit 38 zugeführt wird, ist über ein Dimmer oder Potentiometer 41 regulierbar (siehe Fig. 2).

Damit die Funduskamera 30 und das mit der Funduskamera verbundene Funduskameraobjektiv 20 einfacher und auch nur mit einer Hand zu bedienen sind, ist vorzugsweise ein Handgriff 35 vorgesehen, der im Bereich der Kameraanschlusseinheit 36 und/oder der Funduskameraobjektivanschlusseinheit 31 abgestützt ist. Dabei kann der Handgriff 35 über eine nicht näher spezifizierte Verbindungseinheit am Außenumfang des Funduskameraobjektivs befestigt sein. Der Handgriff 35 weist vorzugsweise einen Auslöser 33 auf, der über eine Verbindung 40 mit der Kamera verbunden ist und ein Auslösen der Aufnahme ermöglicht. Weiter ist es möglich, in dem Handgriff 35 Batterien bzw. Akkumulatoren 34 anzuordnen, die eine Stromversorgung beispielsweise der Kamera 30, der externen Beleuchtungsquelle 17, 38 und/oder des Blitzlichts 32 zur Verfügung stellen. Die Auslösung der Aufnahme kann alternativ auch über ein mechanisches Gestänge oder einen Drahtauslöser erfolgen. Die Übertragung des Auslösebefehls kann auch per Funk zur Kamera 30 übertragen werden. In den Handgriff 35 kann auch ein Potentiometer 41a eingebaut werden, mit dem die Beleuchtungsstärke des Ringlichts 18 eingestellt werden kann. Darüber hinaus können im Handgriff 35 zusätzliche Bedienelemente zum Scharfstellen und/oder zur Bildverarbeitung angeordnet werden. Der Handgriff 35 kann auch ohne das Funduskameraobjektiv 20 verwendet werden, wenn beispielsweise ein schweres Teleobjektiv an die Analog- oder Digitalkamera 30 angeschlossen wird. Durch die Verwendung des Handgriffs 35 lässt sich die Kamera mit dem schweren Teleobjektiv leichter handhaben, so dass ein Halten der Kamera auch einhändig möglich ist.

Das Ringlicht 18 kann auch ohne Funduskameraobjektiv 20 eingesetzt werden. Dies ist in Fig. 3 dargestellt. Das Ringlicht 18 ist gemäß Fig. 3 unmittelbar an der Kameraanschlusseinheit 36 angesetzt, um eine Beleuchtung beispielsweise bei einer Aufnahme des Auges zu verbessern. Die Kamera 30 ist mit einem allgemeinen Objektiv 42 an der Kameraanschlusseinheit 36 verbunden. Bei Makroaufnahmen mit Digitalkameras kann der integrierte Kamerablitz 32 meist nicht verwendet werden, da die Beleuchtung während der Makroaufnahme nicht ausreichend ist und durch den seitlichen Versatz des Blitzlichts 32 zum Objektiv 42 das zu fotografierende Objekt nicht mehr ausreichend beleuchtet wird. Um eine ausreichende Beleuchtung zu ermöglichen, kann das Ringlicht 18 somit auch ohne Funduskameraobjektiv eine Beleuchtung eng um das aufzunehmende Objekt herum ermöglichen. Mit einem entsprechenden Aufsatz auf dem Funduskameraobjektiv 20 oder auch einem allgemeinen Objektiv 42 kann die Kamera auch zum Fotografieren von Hohlräumen, wie Ohren, Nasen oder Rachen verwendet werden, wobei der Aufsatz dann je nach Anwendung in seiner Form ausgestaltet ist. Ebenso ist mit einem entsprechend ausgestaltetem Aufsatz ein Aufnehmen von Bildern der Haut und deren Veränderungen möglich. Auch hier sorgt das Ringlicht für ausreichende Beleuchtung im Makrobereich.

In Fig. 3 ist der Handgriff 35 an der Kameraanschlusseinheit 36 bzw. am Objektiv 42 abgestützt. Der Handgriff 35 enthält hier eine Beleuchtungseinheit 38, die über ein Lichtleitkabel 22a Licht zum Ringlicht 18 leitet. Über das Anschlusskabel 40 ist der Handgriff 35 mit der Kamera 30 verbunden, um Befehle der Bedienungselemente 33, 41a etc im Handgriff 35 zur Kamera 30 zu übertragen. Alternativ ist es möglich, den Handgriff 35 ausschließlich an der Kamera 30 abzustützen.

In Fig. 2 ist eine alternative Ausführungsform dargestellt, die auf der Ausführungsform gemäß Fig. 1 beruht. Hierbei ist das Funduskameraobjektiv 20 zusammen mit einer Beleuchtungseinheit 38 und dem Ringlicht 18 so ausgestaltet, dass eine Angiographieaufnahme der Blutgefäße der Netzhaut des Auges 4 möglich ist. Dazu ist es einerseits erforderlich, dass ein von der Beleuchtungseinheit 38 abgestrahltes Licht über einen Erregerfilter 43 gefiltert wird, so dass nur blaues Licht, beispielsweise mit einer Wellenlänge von 480 nm in den Lichtleiter 22a eintritt und über das Ringlicht 18 ausgestrahlt wird. Dem Patienten wird vor der Angiographieaufnahme ein Kontrastmittel, beispielsweise Floureszein, in die Armvene gespritzt, wobei das von der Beleuchtungseinheit 38 ausgestrahlte und vom Erregerfilter 43 gefilterte blaue Licht über den Lichtleiter 22a zum Ringlicht 18 geführt wird, so dass das blaue Licht in das Auge eintritt. Durch das blaue Licht werden die Floureszein-Moleküle angeregt, so dass ein grünes Licht ausgesendet wird. Es wird jedoch auch blaues Licht in Richtung Funduskameraobjektiv 20 reflektiert. Das blaue und grüne Licht trifft dann auf einen Sperrfilter 37, der als Aufsetzfilter vor dem Objektiv 22 angeordnet ist. Der Sperrfilter 37 filtert das reflektierte blaue Licht heraus, so dass nur das grüne Licht in das Funduskameraobjektiv 20 eindringen kann und somit von der Kamera 30 aufgenommen werden kann. Das grüne Licht hat dabei eine Wellenlänge von ca. 510 bis 530 nm.

Alternativ kann der Sperrfilter 37 auch innerhalb des Funduskameraobjektivs 20 angeordnet sein. Die Realisierung als Aufsetzfilter hat jedoch den Vorteil, dass das Funduskameraobjektiv 20 somit auch an andersartige Aufnahmen und an andere Anwendungen angepasst werden kann.

Die Beleuchtungseinheit 38 ist über einen Dimmer bzw. Potentiometer 41 in ihrer Beleuchtungsstärke einstellbar. Weiter ist die Beleuchtungseinheit 38 je nach Anwendung mit einer internen Lichtquelle und/oder einer Blitzröhre versehen, und benötigt somit keinen Strahlenteiler.

Bei der Verwendung der Beleuchtungseinheit 38 mit einer Lichtquelle, die neben der Dimmbarkeit auch Blitzen kann, ist ein Strahlenteiler 21, wie im Ausführungsbeispiel gemäß Fig. 1, nicht notwendig. Das notwendige Blitzlicht zur Ausleuchtung des Augenhintergrundes während der Aufnahme übernimmt dann die Lichtquelle in der Beleuchtungseinheit 38 statt des Kamerablitzes.

Ebenso ist es möglich, diese Beleuchtungseinheit 38 direkt in den Handgriff 35 zu integrieren und somit dem Auge über dieses Ringlicht 18 das erforderliche Licht zuzuführen, um hochqualitative Aufnahmen zu gewährleisten. Die Beleuchtungseinheit 38 kann auch mit Lichtquellen versehen sein, die Licht verschiedener Wellenlänge ausstrahlen. Wenn die Beleuchtungseinheit 38 in der Lage ist, blaues Licht auszustrahlen, ist beispielsweise der Einsatz des Erregerfilters 43 nicht notwendig, da das blaue Licht über den Lichtleiter 22a direkt in das Ringlicht 18 eingekoppelt wird, um somit blaues Licht zum Anregen der Floureszein-Moleküle auszustrahlen.

Die Umrüstung einer Funduskamera mit dem Funduskameraobjektiv 20 gemäß Fig. 2 ist auch für die Ausgestaltung gemäß Fig. 1 möglich.

Mit dem erfindungsgemäßen Funduskameraobjektiv ist somit der Austausch von einzelnen Komponenten möglich, wodurch sich die Kamera ohne weiteres für verschiedene Anwendungsfälle umrüsten lässt. Darüber hinaus ist es möglich, das Funduskameraobjektiv 20 an verschiedene Kameras anzuschließen, um somit den Entwicklungsfortschritt bei analogen bzw. digitalen Kameras auch bei der Verwendung für Augenuntersuchungen einzusetzen, ohne dass häufig teure und komplexe Spezialfunduskameras gekauft werden müssen, bei denen sich die einzelnen Komponenten nicht austauschen lassen.

Mit Hilfe des erfindungsgemäßen Funduskameraobjektivs kann eine herkömmliche Analog- und Digitalkamera zu einer Funduskamera aufgerüstet werden. Dadurch ergibt sich eine kompakte Bauweise und darüber hinaus ein geringes Gewicht, wodurch einhändiges Arbeiten möglich ist. Darüber hinaus ist es nicht erforderlich, die Kamera mit Funduskameraobjektiv über ein Kabel oder Leitungen an eine Basisstation zur Bildverarbeitung anzuschließen.

Die erfindungsgemäße handgehaltene Funduskamera kann somit beispielsweise im Veterinärbereich eingesetzt werden, wodurch Aufnahmen in Stallungen oder auf der Weide erleichtert werden, bei denen keine zusätzliche Basisstation oder Verkabelung möglich ist. Durch den Handgriff mit Auslöser ist es möglich, einhändig zu arbeiten. Weiter ist durch die zusätzlichen Bedienelemente, wie Auslöser oder Dimmer am Handgriff 35 eine erleichterte Handhabung möglich.

## Patentansprüche

1. Funduskameraobjektiv zur Aufnahme eines Augenhintergrundes, enthaltend:
- ein Objektiv (22)
- ein Okular (28),
- einen Tubus (19), der in seiner optischen Länge verstellbar ist, um den Abstand zwischen Objektiv (22) und Okular (28) einzustellen; und
wobei das Funduskameraobjektiv (20) kameraseitig eine Funduskameraobjektivanschlusseinheit (31) aufweist und über die Funduskameraobjektivanschlusseinheit (31) mit einer Kamera (30) koppelbar ist,
wobei das Funduskameraobjektiv (20) im vorderen Bereich mit einem Ringlicht (18) verbunden ist, das über einen Lichtleiter (22a) mit einer externen Lichtquelle (17), einer Beleuchtungseinheit (38) und/oder mit einem Strahlenteiler (21) gekoppelt ist, wobei dem Ringlicht (18) Licht einer vorbestimmten Wellenlänge zuführbar ist.

2. Funduskameraobjektiv nach Anspruch 1, **dadurch gekennzeichnet, dass** der Strahlenteiler (21) zwei Eingänge (E1, E2) und wenigstens einen Ausgang (A1, A2) aufweist, wobei der wenigstens eine Ausgang (A1, A2) mit dem Ringlicht (18) koppelbar ist und den Eingängen (E1, E2) Licht einer Lichtquelle (17) und/ oder eines Blitzlichtes (32) zuführbar ist.

3. Funduskameraobjektiv nach Anspruch 1 oder 2, **dadurch gekennzeichnet, dass** der Strahlenteiler (21) mit einem Strahlenumformer (16) verbunden ist, der an seiner einen Seite den Querschnitt eines Blitzlichtes (32) der Kamera (30) oder einer separaten Blitzröhre und an der gegenüberliegenden Seite einen runden Querschnitt mit einer Optik zur Fokussierung auf einen Querschnitt des Strahlenteilers (21) aufweist.

4. Funduskameraobjektiv nach einem der Ansprüche 1 bis 3, **dadurch gekennzeichnet, dass** das Funduskameraobjektiv (20) mit einem Handgriff (35) koppelbar ist, wobei der Handgriff (35) Batterien (34) oder Akkumulatoren zur Energieversorgung der Kamera (30), des Blitzlichtes (32) und/ oder der Lichtquelle (17) für das Ringlicht (18) aufweist.

5. Funduskameraobjektiv nach Anspruch 4, **dadurch gekennzeichnet, dass** der abnehmbare Handgriff (35) einen Auslöser (33) aufweist, der mit der Kamera (30) zum Auslösen einer Aufnahme koppelbar ist.

6. Funduskameraobjektiv nach einem der Ansprüche 1 bis 5, **dadurch gekennzeichnet, dass** eine Einstellvorrichtung (24) zum Dioptrienausgleich durch Verschieben von Objektiv (22) und Okular (28) zueinander vorhanden ist, wobei am äußeren Tubus (19) die ausgeglichenen Dioptrien auf einer Skala ablesbar sind.

7. Funduskameraobjektiv nach einem der Ansprüche 1 bis 6, **dadurch gekennzeichnet, dass** im Strahlengang des Funduskameraobjektives (20) eine Feldblende (27), vorzugsweise in Form einer Irisblende, angeordnet ist, wobei der Durchmesser der Feldblende (27) über einen Blendeneinstellring (25) verstellbar ist oder im Strahlengang des Funduskameraobjektives (20) eine Feldblende (27) angeordnet ist, die eine fixe Blende aufweist.

8. Funduskameraobjektiv nach Anspruch 1, **dadurch gekennzeichnet, dass** vor dem Ringlicht (18) ein einsetzbares Erregerfilter (43) angeordnet ist, um Licht einer vorbestimmten Wellenlänge passieren zu lassen, insbesondere blaues Licht mit einer Wellenlänge von ca. 480nm, wobei im Strahlengang des Funduskameraobjektives ein Sperrfilter (37) einsetzbar ist, das insbesondere grünes Licht einer Wellenlänge von ca. 510- 530nm passieren lässt.

9. Funduskameraobjektiv nach Anspruch 1, **dadurch gekennzeichnet, dass** die dem Ringlicht (18) zugeführte Lichtmenge einstellbar ist.

10. Funduskameraobjektiv nach einem der Ansprüche 1 bis 9, **dadurch gekennzeichnet, dass** die Funduskameraobjektivanschlusseinheit (31) ein Gewinde oder einen Bajonettverschluss aufweist.

11. Kamera mit einem Funduskameraobjektiv nach einem der Ansprüche 1 bis 10, wobei die Kamera (30) eine Kameraanschlusseinheit (36) aufweist, die mit der Funduskameraobjektivanschlusseinheit (31) korrespondiert.

12. Kamera nach Anspruch 11, wobei der Strahlenumformer (16) an das Blitzlicht (32) der Kamera (30) anschliessbar ist.

13. Kamera nach Anspruch 11 oder 12, wobei der Handgriff (35) gemäss einem der Ansprüche 5 oder 6 eine Lichtquelle (17, 38) enthält, deren Licht in den Strahlenteiler (21) einkoppelbar ist oder dem Ringlicht (18) direkt zuführbar ist.

14. Handgriff (35), der mit einer Kamera (30) nach einem der Ansprüche 11 bis 13 oder einem Kameraobjektiv (20, 42) nach einem der Ansprüche 1-10 verbindbar ist und Batterien (34) oder Akkumulatoren zur Energieversorgung der Kamera (30), des Blitzlichtes (32) und/oder der Lichtquelle (17) für das Ringlicht (18) und/oder einen Auslöser (33) aufweist, der mit der Kamera (30) zum Auslösen einer Aufnahme koppelbar ist.

## Claims

1. A fundus camera objective for recording an eye fundus, comprising:
- an objective (22),
- an ocular (28),
- a tube (19), the optical length of the tube (19) can be adjusted in order to set the distance between the objective (22) and ocular (28);
wherein the fundus camera objective (20) having a fundus camera objective connection unit (31) on the camera side, and being connectable via the fundus camera objective connection unit (31) to a camera (30),
wherein the fundus camera objective (20) is connected in the front area to a ring light (18), which is coupled via a light guide (22a) to at least one of an external light source (17), an illumination unit (38) and a beam splitter (21), for feeding light of a predetermined wavelength to the ring light (18).

2. The fundus camera objective as claimed in claim 1, wherein the beam splitter (21) has two inputs (E1, E2) and at least one output (A1, A2), the at least one output (A1, A2) being connectable to the ring light (18), wherein light from at least one of a light source (17) and a flashlight (32) is feedable to the inputs (E1, E2).

3. The fundus camera objective as claimed in claims 1 or 2, wherein the beam splitter (21) is connected to a beam shaper (16) that has at one of its ends the cross section of a flashlight (32) of the camera (30) or of a separate flash tube, and at the opposite end a round cross section with an optics for focusing on a cross section of the beam splitter (21).

4. The fundus camera objective as claimed in claims 1 to 3, wherein the fundus camera objective (20) can be coupled to a handle (35), the handle (35) comprises batteries (34) or rechargeable batteries for supplying energy to the camera (30), the flashlight (32) and/or the light source (17) for the ring light (18).

5. The fundus camera objective as claimed in claim 4, wherein a removable handle (35) has a release (33) that can be coupled to the camera (30) in order to trigger a recording.

6. The fundus camera objective as claimed in claims 1 to 5, wherein a setting device (24) is provided for diopter compensation by displacing the objective (22) and ocular (28) relative to one another, it being possible to read out the compensated diopters on a scale on the outer tube (19).

7. The fundus camera objective as claimed in claims 1 to 6, wherein a field diaphragm (27), preferably in the form of an iris diaphragm, is arranged in the beam path of the fundus camera objective (20), the diameter of the field diaphragm (27) being adjustable via a diaphragm setting ring (25) or a field diaphragm (27) having a fixed diaphragm is arranged in the beam path of the fundus camera objective (20).

8. The fundus camera objective as claimed in claim 1, wherein an insertable excitation filter (43) is arranged upstream of the ring light (18) in order to allow passage of light of a predetermined wavelength, in particular blue light with a wavelength of approximately 480 nm, it being possible to insert in the beam path of the fundus camera objective a blocking filter (37) that particularly allows passage of green light with a wavelength of approximately 510-530 nm.

9. The fundus camera objective as claimed in claim 1, wherein the amount of light fed to the ring light (18) can be set.

10. The fundus camera objective as claimed in claims 1 to 9, wherein the fundus camera objective connection unit (31) has a thread or a bayonet lock.

11. A camera having a fundus camera objective as claimed in claims 1 to 10, wherein the camera (30) has a camera connection unit (36) corresponding to the fundus camera objective connection unit (31).

12. The camera as claimed in claim 11, in which a beam shaper (16) can be connected to the flashlight (32) of the camera (30).

13. The camera as claimed in claims 11 or 12, wherein the handle (35) comprises a light source (17, 38) as claimed in claims 5 or 6, whose light can be coupled into the beam splitter (21) or can be fed directly to the ring light (18).

14. A handle (35) that can be connected to a camera (30) as claimed in claims 11 to 13 or a camera objective (20, 42) as claimed in claims 1 to 10, the handle having at least one of the batteries (34) or rechargeable batteries for supplying energy to at least one of the camera (30), the flashlight (32) and the light source (17) for the ring light (18), and a release (33) being connectable to the camera (30) in order to trigger a recording.

## Revendications

1. Objectif de caméra de fond d'oeil pour l'enregistrement d'un fond de l'oeil, comprenant :
- un objectif (22)
- un oculaire (28),
- un tube (19) dont la longueur optique est réglable afin de régler la distance entre l'objectif (22) et l'oculaire (28) ; et
dans lequel l'objectif de caméra de fond d'oeil (20) présente, côté caméra, une unité de raccordement d'objectif de caméra de fond d'oeil (31), et peut être accouplé à une caméra (30) par l'intermédiaire de l'unité de raccordement d'objectif de caméra de fond de l'oeil (31),
l'objectif de caméra de fond d'oeil (20) étant relié, dans la zone antérieure, à un éclairage annulaire (18) qui est accouplé, via un guide de lumière (22a), à une source de lumière (17) externe, une unité d'éclairage (38) et/ou un séparateur de faisceaux (21), moyennant quoi de la lumière d'une longueur d'onde prédéfinie peut être amenée à l'éclairage annulaire (18).

2. Objectif de caméra de fond d'oeil selon la revendication 1, **caractérisé en ce que** le séparateur de faisceaux (21) présente deux entrées (E1, E2) et au moins une sortie (A1, A2), l'au moins une sortie (A1, A2) pouvant être couplée avec l'éclairage annulaire (18), et de la lumière d'une source de lumière (17) et/ou d'un flash (32) pouvant être amenée aux entrées (E1, E2).

3. Objectif de caméra de fond d'oeil selon la revendication 1 ou 2, **caractérisé en ce que** le séparateur de faisceaux (21) est relié à un convertisseur de faisceaux (16) qui présente, sur l'un de ses côtés, la section transversale d'un flash (32) de la caméra (30) ou bien d'un tube-flash distinct et sur le côté opposé, une section transversale ronde avec une optique pour la focalisation sur une section transversale du séparateur de faisceaux (21).

4. Objectif de caméra de fond d'oeil selon l'une des revendications 1 à 3, **caractérisé en ce que** l'objectif de caméra de fond d'oeil (20) peut être accouplé à une poignée (35), la poignée (35) présentant des piles (34) ou des accumulateurs pour l'alimentation en énergie de la caméra (30), du flash (32) et/ou de la source de lumière (17) pour l'éclairage annulaire (18).

5. Objectif de caméra de fond d'oeil selon la revendication 4, **caractérisé en ce que** la poignée (35) amovible présente un déclencheur (33), lequel peut être accouplé à la caméra (30) pour déclencher une prise de vue.

6. Objectif de caméra de fond d'oeil selon l'une des revendications 1 à 5, **caractérisé en ce qu'**il y a un dispositif de réglage (24) pour la compensation dioptrique par décalage l'un par rapport à l'autre de l'objectif (22) et de l'oculaire (28), les dioptries compensées pouvant être lues sur une graduation sur le tube (19) extérieur.

7. Objectif de caméra de fond d'oeil selon l'une des revendications 1 à 6, **caractérisé en ce que**, dans la trajectoire des rayons de l'objectif de caméra de fond d'oeil (20) un diaphragme de champ (27) est disposé, de préférence sous la forme d'un diaphragme iris, moyennant quoi le diamètre du diaphragme de champ (27) peut être réglé grâce à une bague de réglage d'obturateur (25), ou bien **en ce qu'**un diaphragme de champ (27) est disposé dans la trajectoire des rayons de l'objectif de caméra de fond d'oeil (20), lequel présente un diaphragme fixe.

8. Objectif de caméra de fond d'oeil selon la revendication 1, **caractérisé en ce qu'**un filtre d'excitation (43) insérable est disposé devant l'éclairage annulaire (18) afin de laisser passer de la lumière d'une longueur d'onde prédéfinie, en particulier de la lumière bleue avec une longueur d'onde d'environ 480 nm, un filtre d'arrêt (37) pouvant être mis en place dans la trajectoire des rayons de l'objectif de caméra de fond d'oeil, lequel laisse en particulier passer de la lumière verte d'une longueur d'onde d'environ 510-530 nm.

9. Objectif de caméra de fond d'oeil selon la revendication 1, **caractérisé en ce que** la quantité de lumière amenée à l'éclairage annulaire (18) peut être réglée.

10. Objectif de caméra de fond d'oeil selon l'une des revendications 1 à 9, **caractérisé en ce que** l'unité de raccordement d'objectif de caméra de fond d'oeil (31) présente un filetage ou une fermeture à baïonnette.

11. Caméra avec un objectif de caméra de fond d'oeil selon l'une des revendications 1 à 10, la caméra (30) présentant une unité de raccordement de caméra (36), laquelle correspond à l'unité de raccordement d'objectif de caméra de fond d'oeil (31).

12. Caméra selon la revendication 11, dans laquelle le convertisseur de faisceaux (16) peut être raccordé au flash (32) de la caméra (30).

13. Caméra selon la revendication 11 ou 12, dans laquelle la poignée (35) selon l'une des revendications 5 ou 6 contient une source de lumière (17, 38) dont la lumière peut être injectée dans le séparateur de faisceaux (21) ou bien peut être directement amenée à l'éclairage annulaire (18).

14. Poignée (35), laquelle peut être reliée à une caméra (30) selon l'une des revendications 11 à 13 ou à un objectif de caméra (20, 42) selon l'une des revendications 1-10 et qui présente des piles (34) ou des accumulateurs pour l'alimentation en énergie de la caméra (30), du flash (32) et/ou de la source de lumière (17) pour l'éclairage annulaire (18) et/ou un déclencheur (33), lequel peut être accouplé à la caméra (30) pour déclencher une prise de vue.
